# FASCICULE DE BREVET EUROPEEN

(11) **EP 3 854 340 B1**
(45) Date de publication et mention de la délivrance du brevet: **30.10.2024**
(21) Numéro de dépôt: 21315006.3
(22) Date de dépôt: 16.01.2021
(51) Int. Cl.: A61B 34/30, A61B 17/88, A61B 17/70, B25B 23/10

(54) **DISPOSITIF D'AIDE À L'IMPLANTATION D'UNE VIS OSSEUSE DANS LE MILIEU OSSEUX D'UN ÊTRE VIVANT**
HILFSVORRICHTUNG FÜR DIE IMPLANTATION EINER KNOCHENSCHRAUBE IN DAS KNOCHENMEDIUM EINES LEBEWESENS
DEVICE TO ASSIST WITH THE IMPLANTATION OF A BONE SCREW IN THE OSSEOUS MEDIUM OF A LIVING BEING

(30) Priorité: 25.01.2020 FR 2000736
(43) Date de publication de la demande: 28.07.2021
(73) Titulaire: Spinedust, 13008 Marseille (FR)
(72) Inventeur: GRAZIANI, Noël, 13004 Marseille (FR); LEVRIER, Olivier, 13008 Marseille (FR)
(74) Mandataire: Flavenot, Bernard

(56) Documents cités:
- US-A1- 2007 162 046
- US-A1- 2008 269 768
- US-A1- 2014 094 822
- US-A1- 2018 147 018
- US-A1- 2019 090 966

## Description

### Domaine technique

La présente invention concerne les dispositifs d'aide à l'implantation de vis osseuses dans le milieu osseux d'un être vivant, qui trouvent une application particulièrement avantageuse dans le domaine de l'ostéosynthèses rachidienne, plus particulièrement pour l'implantation de systèmes qui permettent le maintien de deux vertèbres, consécutives ou non, l'une par rapport à l'autre en vue de la réalisation d'une arthrodèse rachidienne dans le but par exemple de supprimer la cause des douleurs générées par une fracture de vertèbres ou pour éviter le risque de complication paralytique liée à cette fracture ou encore pour traiter une pathologie dégénérative ou tumorale de la colonne vertébrale.

En effet, les praticiens dans le domaine de la chirurgie rachidienne utilisent par exemple des systèmes comportant essentiellement une plaque ou barre et des vis comportant chacune une tige à filetage osseux terminée par une tête d'épaulement, ces vis étant aptes à coopérer solidairement avec la plaque ou la barre pour que cette plaque ou cette barre soit maintenue en appui contre les vertèbres, emprisonnée entre ces vertèbres et les têtes d'épaulement des vis, dans le but de maintenir solidement les vertèbres pour que se réalise, entre elles, l'ostéosynthèse recherchée.

### ART ANTERIEUR

Ces systèmes d'ostéosynthèse rachidienne connus, comme par exemple ceux décrits dans les documents US 2018/147018, US 2014/094822 et US 2008/269768, donnent de très bons résultats. Mais il est facilement concevable que l'implantation de vis dans la partie osseuse plus particulièrement des vertèbres pose de nombreux problèmes aux Chirurgiens, notamment dus à l'environnement dans ce cas particulier, qui sont inhérents à la structure et à la fonctionnalité de la colonne vertébrale. Ces conditions imposent que le vissage des vis soit effectué avec une très grande précision en position et en profondeur, donc en toute fiabilité, avec le minimum de risque pour le patient.

Le cas du traitement médico-chirurgical donné ci-dessus n'est qu'un exemple. Les problèmes qui se posent avec cet exemple peuvent se retrouver dans d'autres cas de traitements médicaux.

Aussi, la présente invention a-t-elle pour but de réaliser un dispositif d'aide à l'implantation de vis osseuses dans le milieu osseux d'un être vivant, qui tente de limiter au maximum les risques d'une telle implantation en permettant une implantation la plus sécurisée possible, qui soit d'une réalisation la plus simple et la moins onéreuse possible par rapport aux dispositifs similaires connus de l'art antérieur, tout en facilitant le travail des Praticiens.

### Définition de l'invention

Plus précisément, la présente invention a pour objet un dispositif d'aide à l'implantation d'une vis osseuse dans un milieu osseux, conformément à au moins l'une des revendications annexées.

### Brève Description des dessins

D'autres caractéristiques et avantages de la présente invention apparaîtront au cours de la description suivante donnée en regard du dessin annexé à titre illustratif, mais nullement limitatif, dans lequel :
La figure 1 est un schéma de principe, vu en coupe longitudinale, d'un mode de réalisation préféré du dispositif selon l'invention pour une aide à l'implantation d'une vis pédiculaire ou osseuse dans un milieu osseux, et
Les figures 2 et 3 représentent des vues en perspective cavalière d'un mode de réalisation perfectionné d'un des éléments essentiels du dispositif selon la représentation schématique de la figure 1.

### Avertissement

Il est précisé que, dans la présente description, si l'adverbe "sensiblement" est associé à un qualificatif d'un moyen donné, ce qualificatif doit être compris au sens strict ou approché.

### Description d'un mode de réalisation préféré de l'objet de l'invention.

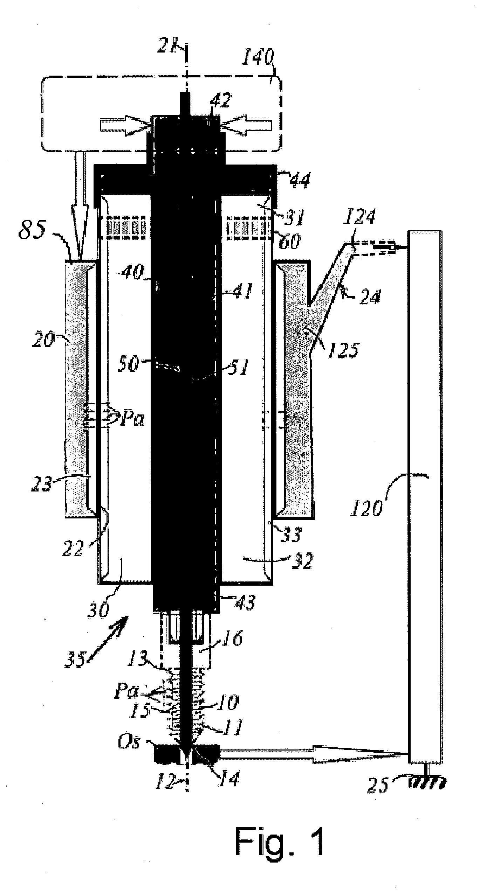

En référence à la figure 1 annexée, l'invention est relative à un dispositif d'aide à l'implantation d'une vis osseuse 10 dans un milieu osseux Os.

Une telle vis comporte une tige de vis 11 définie selon un axe de vissage 12 entre une extrémité proximale 13 et une extrémité distale 14 ; un filetage osseux 15 réalisé à la périphérie de la tige de vis 11, ce filetage osseux ayant un pas de valeur déterminée égale à Pa ; une tête de vis 16 solidaire de l'extrémité proximale 13 de la tige de vis, l'extrémité distale 14 de la tige de vis ayant une configuration lui permettant de pénétrer par auto taraudage dans le milieu osseux Os. De telles vis sont bien connues en elles-mêmes.

Le dispositif selon l'invention comporte un canon 20 définissant un axe longitudinal 21, une traversée 22 cylindrique de révolution réalisée dans le canon 20 selon l'axe longitudinal 21, un taraudage 23 réalisé sur la paroi de la traversée 22, le taraudage 23 ayant un pas de valeur Pa, et des moyens 24 pour fixer le canon 20 à un point de référence 25 lié au milieu osseux Os.

Il comporte aussi une tige mandrin 30 définie entre une extrémité proximale 31 et une extrémité distale 32, la tige mandrin 30 ayant, sur sa surface latérale, au moins une portion de surface cylindrique de révolution sensiblement complémentaire de la traversée 22, un filetage 33 porté par la portion de surface cylindrique de révolution de la tige mandrin 30, dont le pas est lui aussi de valeur Pa, de façon que la tige mandrin 30 soit apte à se visser dans le taraudage 23 du canon 20.

Le dispositif comporte en outre des moyens 35 pour coupler l'extrémité distale 32 de la tige mandrin 30 avec la tête de vis 16 de façon que l'axe 12 de la tige de vis 11 soit confondu avec l'axe longitudinal 21, et des moyens 140 pour commander la rotation de la tige mandrin 30 par rapport au canon 20.

Quant aux moyens 24 pour fixer le canon 20 à un point de référence 25, ils comportent très préférentiellement un bras 124 ou analogue lié fixement au point de référence 25, optionnellement articulé avec un verrouillage simple, et des moyens 125 pour solidariser le bras 124 au canon 20.

Le point de référence 25 peut être constitué d'un point de l'environnement dans lequel se trouve le patient qui doit être traité comme expliqué au préambule de la description. Ce point peut par exemple être lié, de façon directe ou indirecte, à la table d'opération sur laquelle se trouve immobilisé le patient.

Le dispositif comporte un orifice traversant cylindrique 50 réalisé dans la tige mandrin 30, selon l'axe longitudinal 21, et une broche 51 dont la section transversale est au plus égale à la section transversale de l'orifice traversant 50, la broche étant apte à être enfichée par glissement dans cet orifice traversant.

Une telle broche est aussi connue en elle-même. Elle est souvent désignée, par les Praticiens, sous la dénomination « broche de Kirschner ». Ces broches, généralement en acier inoxydable, stérilisées, affûtées, de faible diamètre et lisses, sont largement utilisées en orthopédie pour réduire des fractures.

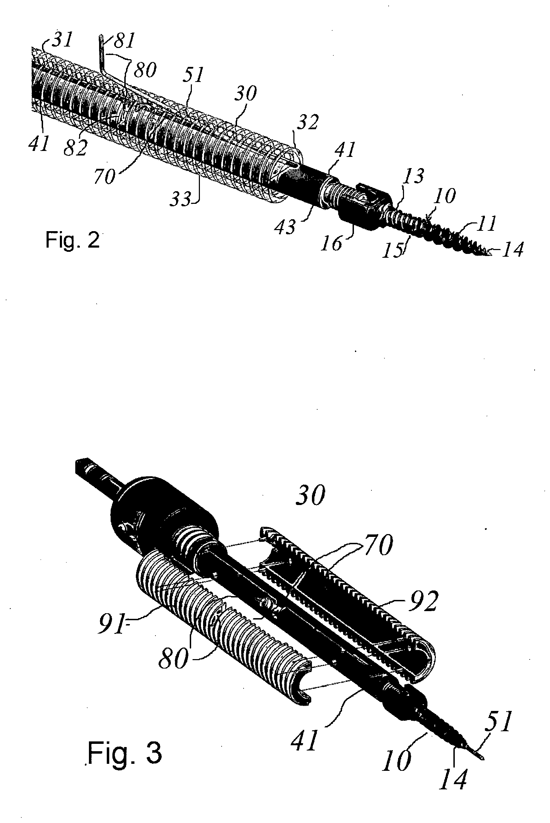

Selon une caractéristique très avantageuse, pour faciliter le montage et surtout l'utilisation du dispositif comme il sera explicité ci-après, la tige mandrin 30 comporte une fenêtre traversante latérale 70, figures 2 et 3, réalisée dans sa paroi, cette fenêtre traversante latérale 70 étant apte à relier l'extérieur de la tige mandrin avec l'orifice traversant 50, la broche 51 étant alors apte à être enfichée dans l'orifice traversant 50 via cette fenêtre traversante latérale du fait de sa souplesse élastique naturelle.

De plus, selon une autre réalisation préférentielle, comme illustré sur les figures, les moyens 35 pour coupler l'extrémité distale 32 de la tige mandrin 30 avec la tête de vis 16 sont constitués très avantageusement par une percée traversante 40 réalisé dans la tige mandrin 30, la percée traversante ayant un axe sensiblement confondu avec l'axe longitudinal 21

Le dispositif comporte alors en outre une tige de liaison 41 définie entre une extrémité proximale 42 et une extrémité distale 43, des moyens 44 pour fixer rigidement et de façon amovible la tige de liaison 41 avec la tige mandrin 30, et des moyens pour lier au moins en rotation et de façon amovible l'extrémité distale 43 de la tige de liaison 41 avec la tête de vis 16, l'orifice traversant 50 étant alors réalisé dans la tige de liaison 41 selon l'axe longitudinal 21.

Les moyens 44 pour fixer rigidement et de façon amovible la tige de liaison 41 avec la tige mandrin 30 peuvent être réalisés de nombreuses façons. A titre d'exemple, ils peuvent être constitués d'un collier ou analogue qui enserre à la fois la tige de liaison 41 et la tige mandrin 30 et de clips qui lient le collier avec respectivement ces deux tiges. Ces éléments sont schématiquement évoqués sur la figure 1.

Selon une caractéristique très préférentielle de l'invention, quand le dispositif, comporte la tige de liaison 41, la fenêtre traversante latérale 70 traverse aussi cette tige de liaison 41.

Selon une réalisation très avantageuse, le dispositif comporte des moyens 80 de fixation en translation de la broche 51 par rapport à la tige mandrin 30 (et la tige de liaison 41 quand elle est présente) après que la broche ait été introduite dans l'orifice traversant 50 via la fenêtre traversante latérale 70.

Ces moyens 80 peuvent être constitués, figure 2, par une déformation de l'extrémité proximale de la broche sous la forme d'une crosse 81, et une encoche latérale 82 réalisée dans la tige mandrin 30 (et la tige de liaison quand elle est présente) dans un plan sensiblement perpendiculaire à l'axe 21, cette encoche débouchant dans la fenêtre traversante latérale 70.

En outre, corrélativement à cette réalisation des moyens 80, le dispositif comporte des moyens pour libérer la fixation de la broche 51 après que la tige mandrin 30 ait été translatée d'une certaine quantité par rapport au canon 20, pour assurer le vissage de la vis osseuse 10 dans un os comme celui d'une vertèbre.

Ces moyens pour libérer la fixation de la broche 51 peuvent être réalisés de nombreuses façons. Ils peuvent même être manuels. Cependant, ils sont avantageusement constitués d'une butée (ou « bump » selon une expression anglaise connue des Praticiens) présente en saillie sur la surface d'extrémité proximale 85 du canon 20, figure 1.

Dans le but d'une sécurité optimale, la crosse 81 peut être soumise à l'action d'un ressort de rappel, comme par exemple une lamelle élastique, pour assurer sa translation de façon plus certaine dans l'encoche 82 pour la ramener dans l'axe de l'orifice traversant 50.

Le dispositif décrit ci-dessus fonctionne de la façon suivante, en précisant que, par « tige mandrin 30 », il faut comprendre pour la description de ce fonctionnement, soit la tige mandrin 30 seule soit en combinaison avec la tige de liaison 41 quand elle est présente, puisque cette dernière est solidaire de la tige mandrin.

Ceci ayant été précisé, la broche 51 est engagée dans la tige mandrin 30 via la fenêtre traversante 70, mais avec la crosse 81 enfichée par rotation dans l'encoche latérale 82 de façon à être verrouillée en position émergente extérieure, l'extrémité distale de la broche dépassant d'une certaine quantité l'extrémité distale 14 de la vis 10, figure 3, par exemple de 15 mm pour un os comme une vertèbre.

Le dispositif est alors actionné et la rotation de la tige mandrin 30 par rapport au canon 20 s'effectue de façon que la vis osseuse 10 vienne tout d'abord au contact de l'os, alors que la broche est déjà rentrée dans l'os de la vertèbre de cette quantité de 15 mm, et se visse dans l'os en suivant la broche déjà implantée dans l'os.

Dans ce mouvement de rotation de la tige mandrin 30 qui fait pénétrer la vis osseuse 10 par vissage dans le milieu osseux, la crosse 81 émergente vient, après un parcours déterminé de la tige mandrin, buter contre la butée présente à la surface d'extrémité 85 du canon 20.

La crosse 81 se désengage alors de l'encoche latérale 82 sous l'effet de la rotation de la tige mandrin 30, ce désengagement étant éventuellement favorisé par la lamelle élastique si elle est présente, et la crosse se retrouve dans l'axe de la fenêtre traversante 70, et donc aussi de la percée 50.

La rotation de la tige mandrin 30 est maintenue, ce qui a pour effet de faire avancer par vissage la vis dans l'os, alors que la broche 51 reste immobile sur son axe puisque bloquée par le canon 20 et ne pénètre pas davantage dans l'os, d'où la sécurité de ne pas atteindre des organes vitaux du patient, la position de la broche 51 pouvant d'ailleurs être vérifiée, par sécurité supplémentaire, via un guidage radiologique.

Selon une autre réalisation avantageuse pour son utilisation, figure 3, la tige mandrin 30 est constituée d'au moins deux coques 91, 92 aptes à être assemblées fixement l'une avec l'autre mais tout en laissant un espace entre deux bords longitudinaux opposés de ces deux coques, pour que cet espace constitue la fenêtre traversante latérale 70 définie ci-avant.

Un des avantages de cette réalisation est de permettre l'utilisation du dispositif avec des vis de différents diamètres sans avoir à changer l'ensemble des éléments constitutifs du dispositif.

De façon préférentielle, les moyens pour lier au moins en rotation et de façon amovible l'extrémité distale 43 de la tige de liaison 41 avec la tête de vis 16 sont constitués par des moyens encliquetables/décliquetables, dont la réalisation est bien connue en elle-même des hommes du métier, par exemple de type à cliquets à contrainte élastique.

Selon une réalisation avantageuse et préférée, le dispositif comporte en outre des moyens pour déterminer la position de la tige mandrin 30 par rapport au canon 20. Ces moyens sont par exemple constitués par une bague 60 solidaire à la périphérie de la tige mandrin 30 et comportant des repères fiduciaires pour être aptes à coopérer avec un système de triangulation. La tige mandrin 30 est alors positionnée par un système de navigation faisant correspondre les repères fiduciaires détectés par un système de triangulation externe, à une base de données d'imagerie 3D du patient (scanner, IRM).

Selon une réalisation très avantageuse, pour réduire les efforts à appliquer sur la tige mandrin lors du vissage de la vis pédiculaire, par notamment auto-taraudage dans un milieu osseux, comme cela se fait couramment en matière de chirurgie, le diamètre de la tige mandrin 30 (son filetage 33 non compris) est supérieur au diamètre du cylindre fictif enveloppant le filetage 15 de la tige de vis 11.

En outre, pour faciliter l'utilisation du dispositif, la longueur de la tige mandrin 30 est supérieure à la longueur du canon 20.

L'utilisation du dispositif selon l'invention se déduit sans difficulté de la description faite ci-dessus.

L'utilisation du dispositif selon l'invention tel que décrit ci-dessus permet à l'évidence de réduire encore plus, par rapport aux dispositifs similaires de l'art antérieur, les risques de l'implantation d'une vis osseuse, en optimisant les conditions physiques de cette implantation, notamment le maintien de l'axe de vissage de la vis.

La structure de ce dispositif permet en outre de l'adapter à chaque type de tête de vis, à condition cependant que les vis aient le même pas de vis Pa que ceux du taraudage 23 et du filetage 33, en n'interchangeant que la tige de liaison 41, sans devoir changer la tige mandrin 30.

## Revendications

1. Dispositif d'aide à l'implantation d'une vis osseuse (10) dans un milieu osseux (Os), ladite vis comportant : une tige de vis (11) définie selon un axe de vissage (12) entre une extrémité proximale (13) et une extrémité distale (14) ; un filetage osseux (15) réalisé à la périphérie de ladite tige de vis (11), ledit filetage osseux ayant un pas de valeur Pa ; une tête de vis (16) solidaire de l'extrémité proximale (13) de la tige de vis, l'extrémité distale (14) de la tige de vis ayant une configuration lui permettant de pénétrer par auto taraudage dans le milieu osseux (Os), ledit dispositif comportant :
• un canon (20) définissant un axe longitudinal (21),
• une traversée (22) cylindrique de révolution réalisée dans ledit canon (20) selon ledit axe longitudinal (21),
• un taraudage (23) réalisé sur la paroi de ladite traversée (22), ledit taraudage (23) ayant un pas de valeur Pa,
• des moyens (24) pour fixer ledit canon (20) à un point de référence (25) lié audit milieu osseux (Os),
• une tige mandrin (30) définie entre une extrémité proximale (31) et une extrémité distale (32), ladite tige mandrin (30) ayant, sur sa surface latérale, au moins une portion de surface cylindrique de révolution sensiblement complémentaire de ladite traversée (22),
• un filetage (33) porté par ladite portion de surface cylindrique de révolution de la tige mandrin (30), dont le pas est lui aussi de valeur Pa, de façon que ladite tige mandrin (30) soit apte à se visser dans le taraudage (23) dudit canon (20),
• des moyens (35) pour coupler l'extrémité distale (32) de la tige mandrin (30) avec la tête de vis (16) de façon que l'axe (12) de la tige de vis (11) soit confondu avec l'axe longitudinal (21), et
• des moyens (140) pour commander la rotation de ladite tige mandrin (30) par rapport audit canon (20),
**caractérisé par le fait qu'**il comporte en outre :
• un orifice traversant cylindrique (50) réalisé dans ladite tige mandrin (30), selon ledit axe longitudinal (21), et
• une broche (51) dont la section transversale est au plus égale à la section transversale dudit orifice traversant (50), ladite broche étant apte à être enfichée dans ledit orifice traversant (50).

2. Dispositif selon la revendication 1, **caractérisé par le fait que** ladite tige mandrin (30) comporte une fenêtre traversante latérale (70) réalisée dans sa paroi, cette fenêtre traversante latérale (70) étant apte à relier l'extérieur de la tige mandrin avec ledit orifice traversant (50), ladite broche (51) étant apte à être enfichée dans ledit orifice traversant (50) via ladite fenêtre traversante latérale.

3. Dispositif selon l'une des revendications 1 et 2, **caractérisé par le fait que** les moyens (35) pour coupler l'extrémité distale (32) de la tige mandrin (30) avec la tête de vis (16) sont constitués par :
• une percée traversante (40) réalisée dans ladite tige mandrin (30), ladite percée traversante ayant un axe sensiblement confondu avec ledit axe longitudinal (21),
• une tige de liaison (41) définie entre une extrémité proximale (42) et une extrémité distale (43),
• des moyens (44) pour fixer rigidement et de façon amovible ladite tige de liaison (41) avec ladite tige mandrin (30), et
• des moyens pour lier au moins en rotation et de façon amovible l'extrémité distale (43) de ladite tige de liaison (41) avec la tête de vis (16),
• ledit orifice traversant (50) étant réalisé dans ladite tige de liaison (41) selon ledit axe longitudinal (21).

4. Dispositif selon la revendication 3 quand elle dépend de la revendication 2, **caractérisé par le fait que** ladite fenêtre traversante latérale (70) traverse aussi ladite tige de liaison (41).

5. Dispositif selon l'une des revendications 2, 4, ou 3 quand elle dépend de la revendication 2, **caractérisé par le fait qu'**il comporte des moyens (80) de fixation en translation de ladite broche par rapport à la tige mandrin (30) après que ladite broche ait été introduite dans ledit orifice traversant (50) via ladite fenêtre traversante latérale (70).

6. Dispositif selon la revendication 5, **caractérisé par le fait qu'**il comporte des moyens pour libérer la fixation de ladite broche (51), après que la tige mandrin (30) ait été translatée d'une certaine quantité par rapport audit canon (20).

7. Dispositif selon l'une des revendications 5 et 6, **caractérisé par le fait que** les moyens (80) de fixation en translation de la broche par rapport à la tige mandrin (30) sont constitués par une déformation de l'extrémité proximale de la broche sous la forme d'une crosse (81) et d'une encoche latérale (82) réalisée dans la tige mandrin (30) et dans la tige de liaison quand elle est présente, ladite encoche latérale étant définie dans un plan sensiblement perpendiculaire à l'axe (21) et débouchant dans ladite fenêtre traversante latérale (70).

8. Dispositif selon l'une des revendications 2 et 4, **caractérisé par le fait que** ladite tige mandrin (30) est constituée d'au moins deux coques (91, 92) aptes à être assemblées l'une avec l'autre tout en laissant un espace entre deux bords longitudinaux opposés de ces deux coques, ledit espace constituant ladite fenêtre traversante latérale (70).

9. Dispositif selon l'une des revendications précédentes, **caractérisé par le fait que** les moyens (24) pour fixer ledit canon (20) à un point de référence (25) comportent au moins :
• un bras (124) lié fixement audit point de référence (25), et
• des moyens (125) pour solidariser ledit bras (124) avec ledit canon (20).

10. Dispositif selon l'une des revendications précédentes quand elle dépend de la revendication 3, **caractérisé par le fait que** les moyens pour lier au moins en rotation et de façon amovible l'extrémité distale (43) de la tige de liaison (41) avec la tête de vis (16) sont constitués par des moyens encliquetables/décliquetables.

11. Dispositif selon l'une des revendications précédentes, **caractérisé par le fait qu'**il comporte en outre des moyens pour déterminer la position de ladite tige mandrin (30) par rapport au canon (20).

12. Dispositif selon la revendication 11, **caractérisé par le fait que** les moyens pour déterminer la position de ladite tige mandrin (30) par rapport au canon (20) sont constitués par une bague (60) solidaire à la périphérie de ladite tige mandrin (30) et comportant des repères fiduciaires aptes à coopérer avec un système de triangulation.

13. Dispositif selon l'une des revendications précédentes, **caractérisé par le fait qu'**il comporte la vis osseuse (10) et que le diamètre de ladite tige mandrin (30), son filetage (33) non compris, est supérieur au diamètre du cylindre fictif enveloppant le filetage (15) de la tige de vis (10) apte à être implantée.

14. Dispositif selon l'une des revendications précédentes, **caractérisé par le fait que** la longueur de la tige mandrin (30) est supérieure à la longueur du canon (20).

## Patentansprüche

1. Vorrichtung als Hilfe für die Implantation einer Knochenschraube (10) in ein Knochenmedium (Os), wobei die Schraube umfasst: einen Schraubenschaft (11), der entlang einer Schraubachse (12) zwischen einem proximalen Ende (13) und einem distalen Ende (14) gebildet ist; ein Knochengewinde (15), das am Umfang des Schraubenschafts (11) ausgebildet ist, wobei das Knochengewinde eine Steigung mit dem Wert Pa hat; einen Schraubenkopf (16), der mit dem proximalen Ende (13) des Schraubenschafts fest verbunden ist, wobei das distale Ende (14) des Schraubenschafts eine Konfiguration aufweist, die es ihm ermöglicht, durch Selbstgewindeschneiden in das Knochenmedium (Os) einzudringen, wobei die Vorrichtung umfasst:
- einen Lauf (20), der eine Längsachse (21) definiert,
- eine rotationszylindrische Durchführung (22), die in dem Lauf (20) entlang der Längsachse (21) ausgeführt ist,
- ein Gewinde (23), das an der Wandung der Durchführung (22) ausgebildet ist, wobei das Gewinde (23) eine Steigung mit dem Wert Pa hat,
- Mittel (24) zum Befestigen des Laufs (20) zu einem Referenzpunkt (25), der mit dem Knochenmedium (Os) in Verbindung steht,
- eine Lochdornstange (30), die zwischen einem proximalen Ende (31) und einem distalen Ende (32) gebildet ist, wobei die Lochdornstange (30) an ihrer Seitenfläche mindestens einen rotationszylindrischen Oberflächenabschnitt aufweist, der im Wesentlichen komplementär zu der Durchführung (22) ist,
- ein Gewinde (33), das von dem rotationszylindrischen Oberflächenabschnitt der Lochdornstange (30) getragen wird, dessen Steigung ebenfalls den Wert Pa hat, so dass die Lochdornstange (30) geeignet ist, in das Innengewinde (23) des Laufs (20) eingeschraubt zu werden,
- Mittel (35), um das distale Ende (32) der Lochdornstange (30) mit dem Schraubenkopf (16) zu koppeln, so dass die Achse (12) des Schraubenschafts (11) mit der Längsachse (21) zusammenfällt, und
- Mittel (140) zum Steuern der Drehung der Lochdornstange (30) in Bezug auf den Lauf (20),
**gekennzeichnet dadurch, dass** ferner vorgesehen sind:
- eine zylindrische Durchgangsöffnung (50), die in der Lochdornstange (30) entlang der Längsachse (21) ausgebildet ist, und
- ein Stift (51), dessen Querschnitt höchstens gleich dem Querschnitt der Durchgangsöffnung (50) ist, wobei der Stift dazu geeignet ist, in die Durchgangsöffnung (50) eingesteckt zu werden.

2. Vorrichtung nach Anspruch 1, **dadurch gekennzeichnet, dass** die Lochdornstange (30) ein seitliches Durchgangsfenster (70) aufweist, das in ihrer Wandung ausgebildet ist, wobei das seitliche Durchgangsfenster (70) geeignet ist, die Außenseite der Lochdornstange mit der Durchgangsöffnung (50) zu verbinden, wobei der Stift (51) geeignet ist, über das seitliche Durchgangsfenster in die Durchgangsöffnung (50) eingesteckt zu werden.

3. Vorrichtung nach Anspruch 1 oder 2, **dadurch gekennzeichnet, dass** die Mittel (35) zum Koppeln des distalen Endes (32) der Lochdornstange (30) mit dem Schraubenkopf (16) gebildet sind von:
- einem Durchgangsloch (40), das in der Lochdornstange (30) vorgesehen ist, wobei das Durchgangsloch eine Achse hat, die im Wesentlichen mit der Längsachse (21) zusammenfällt,
- einem Verbindungsstab (41), der zwischen einem proximalen Ende (42) und einem distalen Ende (43) definiert ist,
- Mitteln (44) zum starren und lösbaren Befestigen des Verbindungsstabs (41) mit der Lochdornstange (30), und
- Mittel, um das distale Ende (43) des Verbindungsstabs (41) zumindest drehbar und lösbar mit dem Schraubenkopf (16) zu verbinden,
- wobei die Durchgangsöffnung (50) in dem Verbindungsstab (41) entlang der Längsachse (21) ausgebildet ist.

4. Vorrichtung nach Anspruch 3, wenn sie von Anspruch 2 abhängt, **dadurch gekennzeichnet, dass** das seitliche Durchgangsfenster (70) ferner durch den Verbindungsstift (41) verläuft.

5. Vorrichtung nach einem der Ansprüche 2, 4 oder 3 und wenn sie von Anspruch 2 abhängt, **dadurch gekennzeichnet, dass** sie Mittel (80) zur translatorischen Fixierung des Stifts in Bezug auf die Lochdornstange (30) umfasst, nachdem der Stift über das seitliche Durchgangsfenster (70) in die Durchgangsöffnung (50) eingeführt worden ist.

6. Vorrichtung nach Anspruch 5, **dadurch gekennzeichnet, dass** sie Mittel zum Lösen der Befestigung des Stifts (51) umfasst, nachdem die Lochdornstange (30) um einen bestimmten Betrag in Bezug auf den Lauf (20) translatorisch bewegt wurde.

7. Vorrichtung nach einem der Ansprüche 5 oder 6, **dadurch gekennzeichnet, dass** die Mittel (80) zur translatorischen Fixierung des Stifts in Bezug auf die Lochdornstange (30) durch eine Verformung des proximalen Endes des Stifts in Form eines Kreuzes (81) und einer seitlichen Einkerbung (82) gebildet werden, die in der Lochdornstange (30) und im Verbindungsschaft, wenn er vorhanden ist, ausgeführt ist, wobei die seitliche Einkerbung in einer Ebene definiert ist, die im Wesentlichen senkrecht zur Achse (21) verläuft und in das seitliche Durchgangsfenster (70) mündet.

8. Vorrichtung nach einem der Ansprüche 2 und 4, **dadurch gekennzeichnet, dass** die Lochdornstange (30) aus mindestens zwei Schalen (91, 92) besteht, die geeignet sind, miteinander verbunden zu werden, wobei zwischen zwei gegenüberliegenden Längsrändern dieser beiden Schalen ein Zwischenraum verbleibt, wobei der Zwischenraum das seitliche Durchgangsfenster (70) bildet.

9. Vorrichtung nach einem der vorhergehenden Ansprüche, **dadurch gekennzeichnet, dass** die Mittel (24) zum Befestigen des Laufs (20) zu einem Referenzpunkt (25) mindestens umfassen:
- einen Arm (124), der fest mit dem Referenzpunkt (25) verbunden ist, und
- Mittel (125), um den Arm (124) fest mit dem Lauf (20) zu verbinden.

10. Vorrichtung nach einem der vorhergehenden Ansprüche, wenn sie von Anspruch 3 abhängt, **dadurch gekennzeichnet, dass** die Mittel zum zumindest drehbaren und lösbaren Verbinden des distalen Endes (43) der Verbindungsstange (41) mit dem Schraubenkopf (16) aus einrastbaren/ausrastbaren Mitteln bestehen.

11. Vorrichtung nach einem der vorhergehenden Ansprüche, **dadurch gekennzeichnet, dass** sie weiterhin Mittel zur Bestimmung der Position der genannten Lochdornstange (30) in Bezug auf den Lauf (20) umfasst.

12. Vorrichtung nach Anspruch 11, **dadurch gekennzeichnet, dass** die Mittel zur Bestimmung der Position der genannten Lochdornstange (30) in Bezug auf den Lauf (20) aus einem Ring (60) bestehen, der fest mit dem Umfang der genannten Lochdornstange (30) verbunden ist und fiduziarische Markierungen aufweist, die geeignet sind mit einem Triangulationssystem zusammenzuwirken.

13. Vorrichtung nach einem der vorhergehenden Ansprüche, **dadurch gekennzeichnet, dass** sie die Knochenschraube (10) umfasst und dass der Durchmesser der genannten Lochdornstange (30), ihr Gewinde (33) nicht eingeschlossen, größer ist als der Durchmesser des fiktiven Zylinders, der das Gewinde (15) des zum Einsetzen geeigneten Schraubenschafts (10) umhüllt.

14. Vorrichtung nach einem der vorhergehenden Ansprüche, **dadurch gekennzeichnet, dass** die Länge der Lochdornstange (30) größer ist als die Länge des Laufs (20).

## Claims

1. Device for assisting with implanting a bone screw (10) into a bone medium (Os), said screw comprising: a screw rod (11) defined along a screwing axis (12) between a proximal end (13) and a distal end (14); a bone thread (15) made at the periphery of said screw rod (11), said bone thread having a pitch of value Pa; a screw head (16) secured to the proximal end (13) of the screw rod, the distal end (14) of the screw rod having a configuration, making it possible for it to penetrate by self-tapping into the bone medium (Os), said device comprising:
• a barrel (20) defining a longitudinal axis (21),
• a cylindrical revolving crossing (22) made in said barrel (20) along said longitudinal axis (21),
• a tapping (23) made on the wall of said crossing (22), said tapping (23) having a pitch of value Pa,
• means (24) for fixing said barrel (20) to a reference point (25) linked to said bone medium (Os),
• a mandrel rod (30) defined between a proximal end (31) and a distal end (32), said mandrel rod (30) having, on its side surface, at least one cylindrical revolving surface portion which is substantially complementary to said crossing (22),
• a thread (33) carried by said cylindrical revolving surface portion of the mandrel rod (30), the pitch of which is itself also of value Pa, such that said mandrel rod (30) is capable of being screwed into the tapping (23) of said barrel (20),
• means (35) for coupling the distal end (32) of the mandrel rod (30) with the screw head (16), such that the axis (12) of the screw rod (11) is merged with the longitudinal axis (21), and
• means (140) for controlling the rotation of said mandrel rod (30) with respect to said barrel (20),
**characterised in that** it further comprises:
• a cylindrical through orifice (50) made in said mandrel rod (30), along said longitudinal axis (21), and
• a wire (51), the cross-section of which is, at most, equal to the cross-section of said through orifice (50), said wire being capable of being plugged into said through orifice (50).

2. Device according to claim 1, **characterised in that** said mandrel rod (30) comprises a side through window (70) made in its wall, this side through window (70) being capable of connecting the outside of the mandrel rod with said through orifice (50), said wire (51) being capable of being plugged into said through orifice (50) via said side through window.

3. Device according to one of claims 1 and 2, **characterised in that** the means (35) for coupling the distal end (32) of the mandrel rod (30) with the screw head (16) are constituted by:
• a through bore (40) made in said mandrel rod (30), said through bore having an axis which is substantially merged with said longitudinal axis (21),
• a connecting rod (41) defined between a proximal end (42) and a distal end (43),
• means (44) for rigidly and removably fixing said connecting rod (41) with said mandrel rod (30), and
• means for linking, at least rotatably and removably, the distal end (43) of said connecting rod (41) with the screw head (16),
• said through orifice (50) being made in said connecting rod (41), along said longitudinal axis (21).

4. Device according to claim 3 when it depends on claim 2, **characterised in that** said side through window (70) also passes through said connecting rod (41).

5. Device according to one of claims 2, 4 or 3, and when it depends on claim 2, **characterised in that** it comprises means (80) for translatably fixing said wire with respect to the mandrel rod (30) after said wire has been introduced into said through orifice (50) via said side through window (70).

6. Device according to claim 5, **characterised in that** it comprises means for releasing the fixing of said wire (51), after the mandrel rod (30) has been translated by a certain quantity with respect to said barrel (20).

7. Device according to one of claims 5 and 6, **characterised in that** the means (80) for translatably fixing the wire with respect to the mandrel rod (30) are constituted by a deformation of the proximal end of the wire in the form of an arch (81) and of a side notch (82) made in the mandrel rod (30) and in the connecting rod when it is present, said side notch being defined in a plane which is substantially perpendicular to the axis (21) and opening into said side through window (70).

8. Device according to one of claims 2 and 4, **characterised in that** said mandrel rod (30) consists of at least two shells (91, 92), capable of being fixedly assembled with one another, while leaving a space between two opposite longitudinal edges of these two shells, said space constituting said side through window (70).

9. Device according to any one of the preceding claims, **characterised in that** the means (24) for fixing said barrel (20) to a reference point (25) comprise at least:
• one arm (124) fixedly linked to said reference point (25), and
• means (125) for securing said arm (124) with said barrel (20).

10. Device according to any one of the preceding claims when it depends on claim 3, **characterised in that** the means for linking at least rotatably and removably, the distal end (43) of the connecting rod (41) with the screw head (16) are constituted by means which can be snap-fitted/disengaged.

11. Device according to any one of the preceding claims, **characterised in that** it further comprises means for determining the position of said mandrel rod (30) with respect to the barrel (20).

12. Device according to claim 11, **characterised in that** the means for determining the position of said mandrel rod (30) with respect to the barrel (20) are constituted by a ring (60) secured to the periphery of said mandrel rod (30) and comprising visible markers capable of engaging with a triangulation system.

13. Device according to any one of the preceding claims, **characterised in that** it comprises the bone screw (10) and that the diameter of said mandrel rod (30), its thread (33) not included, is greater than the diameter of the dummy cylinder surrounding the thread (15) of the screw rod (10) capable of being implanted.

14. Device according to any one of the preceding claims, **characterised in that** the length of the mandrel rod (30) is greater than the length of the barrel (20).
